# EUROPÄISCHE PATENTSCHRIFT

(11) **EP 0 583 685 B1**
(45) Veröffentlichungstag und Bekanntmachung des Hinweises auf die Patenterteilung: **29.05.1996**
(21) Anmeldenummer: 93112482.0
(22) Anmeldetag: 04.08.1993
(51) Int. Cl.: C07C 231/24

(54) **Verfahren zur Reinigung von Fettsäureamiden**
Process for the purification of fatty acid amides
Procédé de purification d'amides d'acides gras

(30) Priorität: 15.08.1992 DE 4227051
(43) Veröffentlichungstag der Anmeldung: 23.02.1994
(73) Patentinhaber: HOECHST AKTIENGESELLSCHAFT, D-65929 Frankfurt am Main (DE)
(72) Erfinder: Dörpinghaus, Norbert Dr., D-65830 Kriftel (DE); Rittner, Siegbert, Dr., D-64546 Mörfelden (DE)

(56) Entgegenhaltungen:
- DE-A- 1 593 658
- DE-A- 2 355 856
- FR-A- 1 459 655
- US-A- 3 920 523
- DATABASE WPI Week 7111, Derwent Publications Ltd., London, GB; AN 71-19942S & JP-B-46 010 533 (NITO CHEM IND LTD)
- DATABASE WPI Week 7349, Derwent Publications Ltd., London, GB; AN 73-75607U & JP-B-48 040 330 (NIPPON SEIKO CO LTD)

## Beschreibung

Fettsäureamide werden durch die Ammonolyse von Fettsäureestern oder durch die dehydratisierende Amidierung von Fettsäuren hergestellt und enthalten im allgemeinen als Hauptverunreinigungen freie Fettsäuren sowie Nitrile und Verunreinigungen des Ausgangsstoffes.

Seit geraumer Zeit besteht ein Bedarf an gereinigten Fettsäureamiden, die einen möglichst geringen Anteil an freien Fettsäuren und Nitrilen enthalten, keine klebrige oder schmierige Konsistenz besitzen, eine geringe Verfärbung aufweisen (Jod-, Gardner-Farbzahl) und frei sind von ranzigem Geruch.

Die japanische Auslegungsschrift Nr. 46/105 33 (Anmeldenummer 43/36844, Anmelder: Nitto Kagaku Kogyo Kabushiki Kaisha) nennt ein Verfahren zur Reinigung von höheren Fettsäureamiden bei dem die Fettsäureamide einer Vakuumdestillation in Gegenwart von tertiärem Natriumphosphat oder tertiärem Kaliumphosphat unterworfen werden. Mit diesem Verfahren werden Fettsäureamide in Ausbeute von 90 bis 97 % erhalten, die einen Nitrilgehalt von 0,7 bis 3 Gew.-%, ausgehend von einem Nitrilgehalt von 4 Gew.-%, aufweisen. Die Färbung des eingesetzten Rohamids beträgt 6 bis 7 Grad (Gardner-Skala). Nachteilig an diesem Verfahren ist, daß nur Rohamide mit einem geringen Gehalt an färbenden Verunreinigungen eingesetzt werden können und die Produkte einen zu hohen Gehalt an Nitril aufweisen.

Die US-A 39 20 523 offenbart ein Verfahren zur Reinigung neutralisierter Fettsäureamide mit 14 bis 22 Kohlenstoffatomen in der Fettsäure, die als Verunreinigung freie Fettsäuren enthalten und in dem die Fettsäureamide einer Molekulardestillation unter vermindertem Druck unterworfen werden. Die verwendeten Roh- oder ungereinigten Fettsäureamide besitzen im allgemeinen einen Gehalt von 2 bis 7 Gew.-% unumgesetzter Fettsäure, bezogen auf das Reaktionsgemisch, sowie 0,5 bis 1 Gew.-% Nitril.
Eine bevorzugte Ausführungsform des Verfahrens besteht in der Neutralisation eines Reaktionsgemisches, das 2 bis 6 Gew.-% Ölsäure enthält, mit einer alkoholischen Lösung eines Alkalimetallhydroxids oder Natriummethylat in Methanol, wobei ein 6 bis 20%iger Überschuß der Base eingesetzt wird. Nach der Neutralisation wird die alkoholische Lösung durch Destillation entfernt und das neutralisierte Gemisch der Molekulardestillation unterzogen. Der Gehalt an freier Ölsäure im Destillat liegt im Bereich von 0,06 bis 0,6 Gew.-%, bei einem Gehalt an freier Ölsäure des Rohamids von 3,4 Gew.-%. Die Ausbeute an Destillat liegt im Bereich von 70 bis 93,5 Gew.-%. Der Nitrilgehalt bleibt unverändert bei 0,5 bis 1 Gew.-%. Die färbenden Verunreinigungen des Ausgangsstoffes liegen in geringen Mengen vor und erzeugen eine Färbung von 4 bis 8 Grad auf der Gardner-Skala. Das Verfahren führt durch die Vakuumdestillation zu einer Verminderung der Verfärbung der Amide auf weniger als 1 Grad (Gardner-Skala).
Nachteilig an diesem Verfahren ist, daß der Nitrilgehalt nicht reduziert werden kann und daß nur Fettsäureamide mit einem relativ geringen und eng begrenzten Gehalt an freier Ölsäure und einem geringen Gehalt an färbenden Verunreinigungen eingesetzt werden können. Weiterhin ist durch die Verwendung von niederen Alkoholen als Lösungsmittel eine zweistufige Destillation erforderlich. Darüber hinaus muß die Abluftproblematik bei organischen Lösemitteln beherrscht werden.

Aufgabe der Erfindung ist es ein Verfahren zur Verfügung zu stellen, bei dem auch Fettsäureamide mit einem Gehalt von mehr als 6 Gew.-% an freier Ölsäure eingesetzt werden können, das Destillat weniger als 0,4 Gew.-% Nitril und weniger als 0,5 Gew.-% freier Ölsäure beinhaltet, bei dem eine zweistufige Destillation nicht zwingend vorgeschrieben ist und sehr dunkle Fettsäureamide mit einem hohen Gehalt an färbenden Substanzen (Gardner-Farbzahl 15 bis 18) gereinigt werden können.

Gegenstand der Erfindung ist ein Verfahren zur Reinigung von Fettsäureamiden im Gemisch mit freien Fettsäuren und gegebenenfalls weiteren Verunreinigungen umfassend die Verfahrensschritte:
- Neutralisation der Fettsäure durch Zugabe einer wäßrig-alkalischen Lösung und
- anschließende Destillation des neutralisierten Gemisches.

Bei den verwendeten Roh- oder ungereinigten Fettsäureamiden handelt es sich im allgemeinen um ein technisches C₁₄-C₂₂-gesättigtes, ungesättigtes oder mehrfach ungesättigtes Fettsäureamid, wie Ölsäureamid, das ein Gemisch von Fettsäureamiden, vorzugsweise Oleylamid darstellt, jedoch auch etwas gesättigte und ungesättigte C₁₄-C₁₆-Amide sowie C₁₈-mehrfach ungesättigte Amide enthalten kann. Außerdem können andere Fettsäureamide, wie Erucasäure- und Stearinsäureamid verwendet werden. Die Ölsäure- und Stearinsäureamide enthalten im allgemeinen etwas an C₁₆-Fettsäureamide. Die Amide können in einfacher Weise in wirtschaftlichem Ausmaß durch Umsetzung der entsprechenden Fettsäuren mit Ammoniak hergestellt werden. Das erhaltene Reaktionsgemisch enthält einen Nitrilgehalt von weniger als 1 Gew.-%, bevorzugt 0,1 bis 0,5 Gew.-% . Die Fettsäure ist im allgemeinen bis zu 15 Gew.-%, bevorzugt weniger als 10 Gew.-%, besonders bevorzugt 2 bis 6 Gew.-%, enthalten, bezogen auf das Rohamidgemisch. Außerdem können auch schwankende Mengen an färbenden Substanzen vorhanden sein. Diese werden im allgemeinen einfach als Farbkörper bezeichnet. In den eingesetzten Gemischen liegen sie in einer Konzentration vor, daß in dem zu reinigenden Fettsäureamid eine Farbe von mehr als 18 Grad auf der Gardner-Skala erzeugt wird. Der Wassergehalt der zu reinigenden Fettsäureamide liegt üblicherweise unterhalb 5 Gew.-%. Desweiteren können in geringen Mengen, unter 1 Gew.-%, Begleitstoffe, wie Ester, Ketone und/oder Kohlenwasserstoffe, enthalten sein. Ein Rohamidreaktionsgemisch dieser Zusammensetzung eignet sich am besten für das erfindungsgemäße Verfahren zur Verringerung des Säuregehaltes. Das Verfahren führt normalerweise zu einer Verminderung der Verfärbung der Amide auf 1 Grad (Gardner-Skala) bei der zweistufigen und 2 bis 6 Grad bei der einstufigen Destillation. Eine Zunahme des Nitrilgehaltes über denjenigen im Ausgangsmaterial läßt sich nicht feststellen.

Bei der Durchführung des erfindungsgemäßen Verfahrens erfolgt zunächst die Neutralisation des Rohamidgemisches, das bis zu 15 Gew.-% Ölsäure enthalten kann, mit einer wäßrigen Lösung eines Alkalimetall- und/oder Erdalkalimetallhydroxids, wie Natriumhydroxid oder Kalziumhydroxid. Die Art der zur Neutralisation verwendeten Base ist nicht kritisch. Die Versuche wurden zwar auf anorganische Basen beschränkt, jedoch können auch andere Basen, wie organische quarternäre Ammoniumhydroxide, z. B. Tetraethylammoniumhydroxid, Tetramethylammoniumhydroxid, Benzyltrimethylammoniumhydroxid und Benzyltriethylammoniumhydroxid verwendet werden.

Für die Neutralisation der Ölsäure im Rohamidgemisch sollte eine möglichst konzentrierte wäßrig-alkalische Lösung verwendet werden, um die nachträgliche abzutrennende Wassermenge so klein wie möglich zu halten. Gleichzeitig ist es erwünscht, das mit wäßrig-alkalischer Lösung behandelte Rohamidgemisch pumpfähig einzustellen, um es anschließend in eine geeignete Destillationsapparatur einspeisen zu können. Abhängig vom Gehalt an freier Fettsäure und dem gewählten Laugenüberschuß wird üblicherweise eine 5 bis 60 gew.-%ige wäßrig-alkalische Lösung eingesetzt, wobei das Verhältnis zwischen Gewicht des Rohamidgemisches zu wäßriger Lauge im Bereich von 10 : 0,5 bis 3 liegt, bevorzugt 10 : 1, beträgt. Es hat sich gezeigt, daß es von Vorteil ist einen Überschuß an wäßrig-alkalischer Lösung einzusetzen. Der Überschuß an wäßrig-alkalischer Lösung beträgt im allgemeinen 0,2 bis 1,8 Mol, bevorzugt 1,0 Mol, bezogen auf den Gehalt an Ölsäure. Obwohl bereits mit einem 0,2 molaren Überschuß an wäßrig-alkalischer Lösung eine ausreichende Reinigung erzielt werden kann, hat sich in der Praxis am besten ein molarer Überschuß bewährt.

Bei der Neutralisation der Fettsäure wird das Rohamidgemisch aufgeschmolzen, mit wäßriger Lauge versetzt und bei 80 bis 100 °C unter Rühren vermischt. Zusätzlich kann die Schmelze mit ungefähr 0,05 Gew.-% eines Antioxidationsmittels, wie Natriumhypophosphit, Hydrochinon oder 2,6-Di-tert.-butyl-4-methylphenol, versetzt werden.

Im Anschluß an die Neutralisation der Ölsäure erfolgt die Abtrennung des Wassers durch Destillation und die destillative Gewinnung des Fettsäureamids.

Das Entfernen des Wassers und die destillative Gewinnung des Fettsäureamids ist nach einem einstufigen oder zweistufigen Verfahren möglich. Die einstufige Destillation ist von Vorteil wenn der Säuregehalt des zu reinigenden Rohamids zwischen 3 und 4 Gew.-% liegt und der NaOH-Überschuß 1,0 bis 1,2 Mol nicht übersteigt. Bei einem höheren Säuregehalt oder NaOH-Überschuß empfiehlt sich eine zweistufige Destillation.

Bei dem einstufigen Verfahren werden Wasser und Fettsäureamide gleichzeitig einer Destillation unterzogen. Die Destillation erfolgt bevorzugt in einem Dünnschichtverdampfer, z. B. dem ®Normag-Rotafilm-Apparat (System Canzler), wobei das Fettsäureamid gleichzeitig mit dem Wasser, das in der Kühlfalle gesammelt wird, über Kopf abdestilliert.

Bei dem zweistufigen Verfahren werden Wasser und Fettsäureamid nacheinander einer Destillation unterzogen. Die Destillation erfolgt bevorzugt in einem Dünnschichtverdampfer, indem zuerst das Wasser im Grobvakuum (300 bis 400 mbar) bei einer Temperatur im Bereich von 120 bis 140 °C abdestilliert wird und die organischen Bestandteile als Konzentrat im Sumpf gesammelt werden. Anschließend wird im Feinvakuum (0,01 bis 0,1 mbar) das Konzentrat destilliert, wobei das gereinigte Ölsäureamid als Kopfphase erhalten wird. Als Oxidationsschutz wird das Destillat mit etwa 0,05 Gew.-% eines Antioxidationsmittels versetzt.

Bei dem zweistufigen Verfahren ist es von Vorteil zwei Destillationsapparaturen in Reihe zu schalten, wobei eine Reihenschaltung aus zwei Dünnschichtverdampfern oder aus einem Dünnschichtverdampfer, in dem das Wasser abdestilliert wird und einem Kurzwegverdampfer, z. B. ®Normag-Rotafilm-Kurzwegverdampfer, in dem das Fettsäureamid destilliert wird, bestehen kann.

Bei dem Einsatz von stark verunreinigten Produkten (Säuregehalt oberhalb 4 Gew.-%, Gardner-Zahl größer 6) hat sich der Einsatz einer Reihenschaltung aus zwei Dünnschichtverdampfern bewährt.

Das so erhaltene Fettsäureamid fällt in hoher Ausbeute und Reinheit mit einem geringen Nitrilgehalt an. Die Farbe des Destillats beträgt 2 bis 6 bei der einstufigen Destillation bzw. 1 Grad nach der Gardner-Skala bei der zweistufigen Destillation.

Aufgrund des geringen Nitrilgehalts besitzt das Produkt eine vorteilhafte feste und harte Konsistenz und kann entsprechend den Verkaufsanforderungen in konfektionierter Form als hochwertiges Mikrogranulat, in Schuppen oder als gemahlenes Pulver, aber auch einfach als in Formen "eingegossene Ware" bereitgestellt werden.

Ein solches Ölsäureamid findet bevorzugt Anwendung als Entformungsmittel, Gleitmittel bei der Kunststoffolienproduktion, Hydrophobierungsmittel und als Rohstoff für die Kosmetikherstellung.

Ein Vorteil der Erfindung liegt darin, daß ein Rohamidgemisch mit einem hohen Gehalt an freier Fettsäure und färbenden Verunreinigungen eingesetzt werden kann. In der Neutralisationsstufe wird Wasser zum Lösen der Hydroxide benutzt, wodurch eine für organische Lösemittel übliche Abluftreinigung entfällt. Für die destillative Reinigung kann sowohl ein Dünnschicht- als auch ein Kurzwegverdampfer eingesetzt werden. Eine Molekulardestillationsanlage wie in DE-OS-23 55 856 angegeben, ist nicht zwingend erforderlich.
Das erfindungsgemäße Verfahren wird anhand der folgenden Beispiele erläutert:

### Beispiel 1 (einstufige Destillation):

### Versuch A:

200 g Rohölsäureamid werden aufgeschmolzen und mit einer wäßrigen Natronlauge versetzt, die 0,96 g festes NaOH in 20 g Wasser enthält. Die Laugenmenge reicht aus, um die gesamte freie Säure im Rohamid zu neutralisieren und noch einen 0,2 molaren Überschuß zu liefern. Das Rohamidgemisch wird nach Zugabe von 0,1 g Antioxidationsmittel 1 Stunde bei 100 °C gut durchmischt.

Das eingesetzte Rohamidgemisch besitzt folgende Zusammensetzung:

| | |
|---|---|
| prim. Ölsäureamid | 86,7 % |
| Ölsäure und -salz | 2,8 % |
| Ölsäurenitril | < 0,5 % |
| Wasser | 4,6 % |
| Summe aus Ester, Keton und Kohlenwasserstoff | < 1,5 % |
| sonstige Verunreinigungen | 3,6 % |
| Erstarrungspunkt | 70,4°C |
| Gardner-Farbzahl | > 18 Grad |

Die neutralisierte laugenhaltige Rohamidschmelze wird einem Laboratoriums-Dünnschichtverdampfer (System Canzler 0,03 m² Verdampferfläche) zugeführt und bei einer Temperatur auf der Verdampferfläche von 190 °C und einem Druck von 0,01 mbar einer Vakuumdestillation unterworfen. Dabei wird das Ölsäureamid bei einer Temperatur von 90 °C gesammelt und mit Antioxidationsmitteln versetzt. Die Zuspeisung wird dabei auf einer Temperatur von 100 °C gehalten.

Auf die gleiche Weise wie in Versuch A werden 200 g Rohölsäureamid mit 1,60 g (B) und 1,76 g (C, D) festem NaOH, das in 20 g Wasser gelöst wird, behandelt und anschließend im Dünnschichtverdampfer destilliert.

Die Ergebnisse der Ölsäureamidreinigung nach der Destillation sind in Tabelle 1 wiedergegeben.

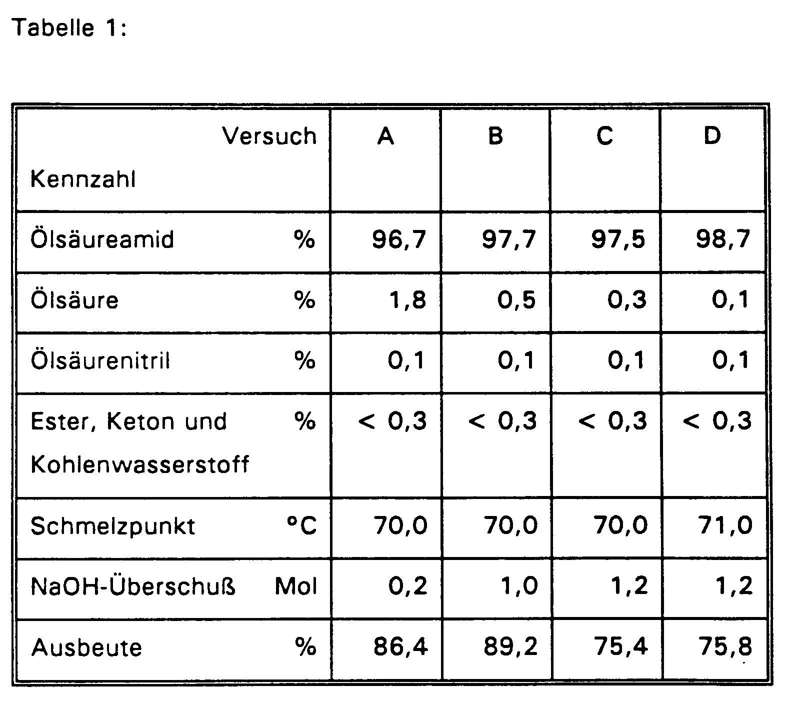

Bei Versuch D wurde der Reinigungsschritt, bestehend aus Neutralisation und Destillation, noch einmal wiederholt. Einsatz war Versuch C. Die Gardner-Farbzahl der Destillate liegt im Bereich von 2 bis 6 Grad.

### Beispiel 2 (zweistufige Destillation):

200 g Rohösäureamid werden mit 8,8 g festem NaOH, gelöst in 20 g Wasser (1,8 molarer Überschuß), der Neutralisation wie in Beispiel 1 unterworfen.

Das verwendete Rohamidgemisch besitzt folgende Zusammensetzung:

| | |
|---|---|
| prim. Ölsäureamid | 87,1 % |
| Ölsäure und -salz | 11,1 % |
| Ölsäurenitril | 0,1 % |
| Wasser | 1,2 % |
| Summe aus Ester, Keton und Kohlenwasserstoff | < 0,3 % |
| Erstarrungspunkt | 63,7°C |
| Gardner-Farbzahl | > 18 Grad |

In einer ersten Verdampferstufe wird das Wasser im Dünnschichtverdampfer bei einer Temperatur von 140 °C und einem Druck von 350 mbar abgetrennt.

Anschließend wird die entwässerte Rohamidschmelze dem Verdampfer wieder zugeführt. Die Amiddestillation erfolgt bei einer Temperatur von 215 °C auf der Verdampferfläche und einem Druck von 0,01 mbar. Die Zuspeisung wird auf 140 °C temperiert, und das Amid bei 90 °C kondensiert. Die Kennzahl des gereinigten Amids gibt Tabelle 2 wieder.

**Tabelle 2**

| Kennzahl | Beispiel 2 |
|---|---|
| Ölsäureamid % | 98,9 |
| Ölsäure % | 1,1 |
| Ölsäurenitril % | 0,1 |
| Ester, Keton u. Kohlenwasserstoff % | < 0,3 |
| Schmelzpunkt °C | 72,0 |
| Jodfarbzahl | 1 |
| Gardner-Farbzahl | 1 |
| APHA-Farbzahl | 123 |
| NaOH-Überschuß Mol | 1,8 |
| Ausbeute % | 71,7 |

## Patentansprüche

1. Verfahren zur Reinigung von Fettsäureamiden umfassend die Verfahrensschritte:
- Neutralisation der Fettsäure durch Zugabe einer wäßrig-alkalischen Lösung und
- anschließende Destillation des neutralisierten Gemisches.

2. Verfahren nach Anspruch 1, dadurch gekennzeichnet, daß die wäßrige Lösung eines Alkalimetallhydroxids, bevorzugt Natriumhydroxid und/oder eines Erdalkalimetallhydroxids, eingesetzt wird.

3. Verfahren nach Anspruch 1 oder 2, dadurch gekennzeichnet, daß eine 5 bis 60 gew.-%ige wäßrig-alkalische Lösung eingesetzt wird.

4. Verfahren nach einem der Ansprüche 1 bis 3, dadurch gekennzeichnet, daß die wäßrig-alkalische Lösung im Überschuß, bevorzugt 0,2 bis 1,8 Mol, bezogen auf den Gehalt an Fettsäure, eingesetzt wird.

5. Verfahren nach einem der Ansprüche 1 bis 4, dadurch gekennzeichnet, daß die Neutralisation durch Zugabe der wäßrig-alkalischen Lösung zur Schmelze des verunreinigten Fettsäureamids und anschließendem Rühren bei einer Temperatur im Bereich von 80 bis 100 °C erfolgt.

6. Verfahren nach Anspruch 5, dadurch gekennzeichnet, daß die Schmelze mit einem Antioxidationsmittel versetzt wird.

7. Verfahren nach einem der Ansprüche 1 bis 6, dadurch gekennzeichnet, daß eine einstufige Destillation, bevorzugt mit einem Dünnschichtverdampfer, erfolgt.

8. Verfahren nach einem der Ansprüche 1 bis 6, dadurch gekennzeichnet, daß eine zweistufige Destillation, bevorzugt Reihenschaltung, besonders bevorzugt Reihenschaltung aus zwei Dünnschichtverdampfern oder einem Dünnschichtverdampfer und einem Kurzwegverdampfer, erfolgt.

## Claims

1. A process for purifying fatty acid amides, comprising the process steps of
- neutralizing the fatty acid by addition of an aqueous alkaline solution and
- subsequently distilling the neutralized mixture.

2. The process as claimed in claim 1, wherein the aqueous solution of an alkali metal hydroxide, preferably sodium hydroxide, and/or of an alkaline earth metal hydroxide is used.

3. The process as claimed in claim 1 or 2, wherein a from 5 to 60% strength by weight aqueous alkaline solution is used.

4. The process as claimed in any one of claims 1 to 3, wherein the aqueous alkaline solution is used in an excess, preferably from 0.2 to 1.8 mol, based on the fatty acid content.

5. The process as claimed in any one of claims 1 to 4, wherein the neutralization is effected by adding the aqueous alkaline solution to the melt of the unpurified fatty acid amide and subsequently stirring at a temperature of from 80 to 100°C.

6. The process as claimed in claim 5, wherein the melt is admixed with an antioxidant.

7. The process as claimed in any one of claims 1 to 6, wherein the distillation is effected in a single stage, preferably using a thin film evaporator.

8. The process as claimed in any one of claims 1 to 6, wherein the distillation is effected in two stages, preferably in series, particularly preferably with an arrangement in series of two thin film evaporators or one thin film evaporator and one flash evaporator.

## Revendications

1. Procédé de purification d'amides d'acides gras, comprenant les étapes selon lesquelles
- on neutralise l'acide gras en ajoutant une solution aqueuse basique et
- on distille ensuite le mélange neutralisé.

2. Procédé selon la revendication 1, caractérisé en ce que l'on utilise une solution aqueuse d'un hydroxyde de métal alcalin, de préférence d'hydroxyde de sodium et/ou d'un hydroxyde de métal alcalino-terreux.

3. Procédé selon la revendication 1 ou 2, caractérisé en ce que l'on utilise une solution aqueuse de base ayant une concentration de 5 à 60 % en masse.

4. Procédé selon l'une des revendications 1 à 3, caractérisé en ce que l'on utilise la solution aqueuse basique en un excès, de préférence de 0,2 à 1,8 moles, par rapport à la teneur en acide gras.

5. Procédé selon l'une des revendications 1 à 4, caractérisé en ce que l'on effectue la neutralisation en ajoutant la solution aqueuse basique à l'amide d'acide gras impur fondu, et en agitant ensuite à une température comprise entre 80 et 100°C.

6. Procédé selon la revendication 5, caractérisé en ce que l'on ajoute un agent antioxydant à la masse fondue.

7. Procédé selon l'une des revendications 1 à 6, caractérisé en ce que l'on effectue une distillation en une étape, de préférence à l'aide d'un évaporateur en couche mince.

8. Procédé selon l'une des revendications 1 à 6, caractérisé en ce que l'on effectue une distillation en deux étapes, de préférence à l'aide d'un montage en série, de façon particulièrement préférée d'un montage en série constitué de deux évaporateurs en couche mince ou d'un évaporateur en couche mince et d'un évaporateur flash.
